# EUROPEAN PATENT SPECIFICATION

(11) **EP 2 482 903 B1**
(45) Date of publication and mention of the grant of the patent: **14.11.2018**
(21) Application number: 10763129.3
(22) Date of filing: 29.09.2010
(51) Int. Cl.: A61K 9/00, A61K 9/12

(54) **IMPROVED METHOD FOR TREATMENT OF PATIENTS WITH CYSTIC FIBROSIS**
VERBESSERTE BEHANLDUNG VON PATIENTEN MIT MUKOVISZIDOSE
TRAITEMENT AMELIORE POUR DES PATIENTS ATTEINTS DE LA MUCOVISCIDOSE

(30) Priority: 29.09.2009 US 246766 P
(43) Date of publication of application: 08.08.2012
(73) Proprietor: Vectura GmbH, 82131 Gauting (DE); Erasmus MC-Sophia Children's Hospital, 3015 GJ Rotterdam (NL)
(72) Inventor: KRONEBERG, Philipp, 82140 Olching (DE); MÜLLINGER, Bernhard, 80634 Munchen (DE); TIDDENS, Harm A. W. N., NL-3066 VA Rotterdam (NL); VAN DEN BEUKEL-BAKKER, Marije, NL-3055 CH Rotterdam (NL)
(74) Representative: Summersell, Richard John
(86) International application number: PCT/EP2010/005944
(87) International publication number: WO 2011/038901

(56) References cited:
- EP-A1- 2 022 525
- WO-A1-95/23613
- WO-A2-2008/039987
- WO-A2-2009/073569
- US-A1- 2007 202 051

## Description

### Background of the Invention

### Field of the Invention

This invention concerns a new and improved treatment of patients with cystic fibrosis by providing an inhalable aerosol comprising dornase alpha administered into a patient's lungs according to a specific treatment protocol comprising a dornase alpha containing aerosol having particles with a predetermined mass medial aerodynamic diameter (MMAD) delivered predominantly to a peripheral airways using a nebulizing system able to administer said aerosol with overpressure and under controlled breathing conditions. The method results in substantial improvement of clinical symptoms of cystic fibrosis patients.

### Background and the Related Disclosures

Cystic fibrosis is one of the most common types of a chronic lung disease in children and young adults that may result in early death. Cystic fibrosis lung disease starts early in life and is progressive. Generally, the children with cystic fibrosis are diagnosed with the disease when they are one or two years old. A milder form of cystic fibrosis appears in patients diagnosed at a later age. However, due to a severity of cystic fibrosis symptoms, exacerbation of the disease may occur in both groups at any time. Screening programs for early diagnosis have been set up in many countries throughout the world.

Cystic fibrosis (CF) is caused by a defective cystic fibrosis gene. Millions of people worldwide, particularly those of Caucasian and Northern or Central European descent, carry the defective CF gene without ever experiencing typical cystic fibrosis symptoms because in order to have cystic fibrosis a person must inherit two defective CF genes, one from each parent.

The defective cystic fibrosis gene directs the body to produce abnormally thick and sticky mucous fluid (mucus). This thick and sticky mucous fluid builds up and accumulates in the breathing passages of the lungs and also in the digestive tract of the cystic fibrosis patient.

The accumulation of the mucus in the lungs results in life-threatening lung and oropharyngeal infections and in serious digestion problems because the mucus also accumulates in the intestines.

The typical cystic fibrosis symptoms are production of thick, viscous mucus secretions in the lungs, repeated infections resulting from the accumulation of mucus in the lungs that create a favorable environment for infectious microorganism, recurrent pneumonia, chronic cough, bronchitis, asthma, chronic sinusitis and nasal polyps.

A major medical problem in most patients with cystic fibrosis, however, is a loss of lung function. The cystic fibrosis patient experiences a gradual worsening of lung function each year due to recurring infections and inflammations. The recurring lung infections often cause permanent scarring of the cystic fibrosis lungs.

In people with cystic fibrosis, loss of lung function is caused by blockage of air passages with infected mucus. The thick mucus plugs the air passages of the lungs resulting in much impaired breathing pattern and shortness of breath.

In order to provide a relief from these serious symptoms, to improve a patient's lung function and to provide a treatment for the disease, a problem of the accumulated and continually accumulating mucus in the lungs must be addressed in such a way that the formation of the mucus is prevented and the accumulated mucus is destroyed and removed from the lungs. Many treatments exist for treatment of symptoms and complications of cystic fibrosis. Their main goal is to prevent infections, reduce amount and thick consistency of mucus secretion and improve the airflow into the lungs.

These treatments include administration of antibiotics, particularly inhalable antibiotics directed to the patient's lungs. However, as is well known, the overuse of antibiotics often leads to a development of bacteria resistant to the antibiotics and also to fungal infections. These treatments also include administration of bronchodilators with the aim to open up bronchial tubes and clear secretions in the airways. Other treatments include a physical removal of the mucus by physiotherapy using manual or mechanical bronchial airway drainage techniques, administered at least twice a day for 20-30 minutes. An option to treat end stage lung disease is lung transplantation that is, of course, a major surgical procedure.

Currently available and promising treatments provide mucus-thinning drugs, mucolytics, which are able to thin the thick mucus by cleaving DNA released from the bacteria in the airways. The treatments with mucolytics used for treating cystic fibrosis lung disease are far from ideal.

The following main problems with these treatments are recognized. First, delivery of these drugs using conventional nebulizers is inefficient, and second, the conventional nebulizers used in the clinical studies for these drugs deliver the mucolytic drug mainly to central airways and not to the lower peripheral airways where the main accumulation of the mucus occurs.

While the treatment with mucolytic drugs is moderately successful in patients with mild to severe cystic fibrosis, it is not so successful in treating the disease during cystic fibrosis exacerbations. During these exacerbations more sputum is produced resulting in great degree and more spread of inflammation obstruction. This is due to the currently available delivery methodology for the mucolytic drugs, such as Pulmozyme® (dornase alpha, RhDNase) administration that results in an uneven distribution of the drug in the lungs. The currently available and used nebulizer administer the drug primarily into the central airways with only a small percentage, if any, of the drug administered into the peripheral airways where the accumulation of the mucus causes a large proportion of the obstruction.

Cystic fibrosis thus presents a serious medical problem and clinical challenge. It would be therefore advantageous to have available reliable treatment of cystic fibrosis that would deal with the mucus formation and accumulation in peripheral and central airways in stable disease and during exacerbations. This can ameliorate serious consequences of this disease and help thousands of patients suffering from cystic fibrosis to reduce or even stop progression of this debilitating life threatening disease.

US 2007/202051 relates to a pharmaceutical aerosol for the treatment of conditions including fibrosis, chronic sinusitis and nasal polyps. The reference discloses the delivery of an active compound such as mucolytics to the mucosa of the nose or of a paranasal sinus comprising a dispersed liquid phase and a continuous gas phase, wherein (a) the volume of the dispersed liquid phase comprising a unit dose of the active compound is less than about 5 mL, (b) the mass median diameter of the dispersed liquid phase is from 2-6 microns and (c) the pressure of the aerosol is maintained at at least 5 m bar.

WO 95/23613 discloses suspension of rhDNase particles for aerosol administration for the treatment of e.g. cystic fibrosis with an aerosolisation device into the lung of an individual wherein the concentration is 0.01-1mg/mg and the particles size 1-6 microns.

WO 2009/073569 describes aqueous formulations comprising a protein at a concentration of at least about 1µg/mL wherein the therapeutic protein is selected from the group consisting of Pulmozyme (Dornase alpha). WO 2009/073569 also describes that proteins (in general) are useful in management of pseudomonas aeruginosa lung infections.

WO 2008/039987 discloses that daily aerosol breathing treatments are very commonly prescribed for CF patients. Aerosolized medicines that can be administered include Pulmozyme.

It is therefore a primary object of this invention to provide a method for efficacious delivery of dornase alpha into the peripheral airways where the drug will cause effective mobilization of sputum and mucus observed to be present in large quantities during stable disease and especially during exacerbations of the disease. The current method provides an efficient delivery of the mucolytic drug into the lung periphery with an AKITA® nebulizing system comprising AKITA® nebulizer, AKITA® compressor and AKITA® protocol wherein the drug is delivered into the peripheral airways with mild to moderate adjustable pressure in an aerosolized form wherein the aerosol has predominantly particle sizes having mass median aerodynamic diameter of about 3 µm. This enables a shift of the mucolytic drug delivery from substantially central deposition to substantially peripheral deposition.

### SUMMARY OF THE INVENTION

One aspect of the current invention is dornase alpha for use in the treatment of cystic fibrosis by administering to a cystic fibrosis patient in need thereof dornase alpha, in an aerosol having a mass medial aerodynamic diameter (MMAD) from about 2 to 6 µm, preferably 3 µm, administered by the AKITA® nebulizing system under mild to moderate pressure substantially into the peripheral airways wherein said aerosol comprises from 1.25 mL to 5 mL (0.75 mg/ml to 1.25 mg/ml, preferably 1 mg/mL) of said dornase alpha of which at least 0.8 mL (0.8 mg) is deposited in the peripheral airways lungs, wherein said nebulizing system is able to administer the aerosolized dornase alpha with high efficiency into the peripheral airways with overpressure of under and up to 30 mbar and wherein said method results in improvement of peripheral flows such as forced expiratory flow rate at 75% (FEF75) of at least 10%, but preferably 45% or more, compared to other conventional nebulizers without side effects. Another aspect of the current invention is a method for delivery of dornase alpha wherein said delivery results in improvement of pulmonary functions, said functions determined by an increase in FEF75 or a forced expiratory volume in one second (FEV1) and by an increased fragmentation of the DNA in the mucus especially in the peripheral airways.

Still another aspect of the current invention is an inhalation method for delivery of dornase alpha wherein a nebulizing system used for such delivery comprises an AKITA® compressor and AKITA® nebulizer in an inhalation system suitable to control a breathing pattern of a patient, wherein a
treatment lasts less than 15 minutes and the drug is administered from one to three times a day in an aerosol having particle sizes of MMAD from about 2 to about 6 µm, preferably predominantly about 3 µm.

Yet another aspect of the current invention is the treatment of a patient with cystic fibrosis requiring a concurrent treatment with mucolytic, comprising administering to the patient in need thereof an inhalable treatment comprising administration of dornase alpha, as an aerosol comprising said drug in amount of 0.75mg/ml to 1.25 mg/ml, preferably 1 mg/mL of from 1.25 mL to 5 mL, wherein said aerosol is administered during an inspiration time comprising three predefined periods, wherein in the first period lasting from 1 millisecond to 1 second, an aerosolized particle free air is administered at a preset flow rate and at a preset volume; wherein in the second period lasting from 0.1 to 7 seconds, aerosolized dornase alpha is administered at a preset flow rate and at a preset volume; wherein in the third period, lasting from 1 millisecond to 10 seconds, an aerosolized particle free air is administered at a preset flow rate and at a preset volume; wherein after the third period, the patient is instructed to stop inhaling and exhale; wherein said protocol is repeated for 6 to 15 minutes or less; and wherein said treatment results in a larger than 0.8 mL deposition of said dornase alpha into the peripheral airways.

Still yet another aspect of the current invention is a treatment of cystic fibrosis wherein the dornase alpha is delivered by an AKITA® nebulizing system equipped with means to preset a flow rate and preset volume for each of the inspirational phases and wherein during an inspirational phase said preset inspirational flow rate is equal or below 20liters/min and wherein an aerosolized particle free air administered in the first period is administered at a preset volume of less than 150 ml in 0.5 second time and wherein said mucolytic aerosol administered in the second period is administered at a volume of from 200 to 2000 mL or in a preset time of from 1 to 7 seconds and wherein said aerosolized particle free air administered in the third period is administered at a preset volume from 200 to 500 mL in 0.3 to 3.0 seconds time.

Another aspect of the current invention is an AKITA® nebulizing protocol suitable for treatment of cystic fibrosis comprising delivering dornase alpha in an aerosol having a particle sizes range limited to particles from 2 to 6 µm MMAD with majority of particles having sizes of 3 µm MMAD, delivered into peripheral airways of the lower lungs using an individualized delivery regimen.

### DEFINITIONS

As used herein:
"MMAD" means mass median aerodynamic diameter.

For determination of particle size distribution and MMAD the Next Generation Impactor should be used at a flow rate through the Impactor of 15 L/min. To reduce evaporation processes the cascade impactor should be cooled to below 10°C by using a cooling cabinet (Marple et al., Journal of Aerosols in Medicine 2004,Vol. 17, No. 4,pp. 335-343; Berg et al., Journal of Aerosols in Medicine 2007, Vol. 20, No. 2, pp. 97-104).

"Mucolytic" or "mucolytic drug" means any drug that is able to fragment, cleave or hydrolyze DNA. An exemplary mucolytic drug is Pulmozyme®, also known as dornase alpha, commercially available as Pulmozyme® from Genentech Corporation, South San Francisco, California. The mucolytic drug may be administered alone or in combination with other mycolytics or other active agents such as antibiotics or corticosteroids.

It should be understood that as used herein the specification of the mucolytic drug in by way of volumetric measures such as "ml" is meant to refer to volumes of the solution or suspension to be aerosolized comprising the mucolytic drug.

"Dornase alpha" or "rhDNase" means a human recombinant deoxyribonucleoside cleaving enzyme. The Pulmozyme® is RhDNase.

"FEF" means forced expiratory flow.

"FVC" means forced vital capacity.

"FEV" means forced expiratory volume.

"FEV1" means forced expiratory volume in one second.

"VC" means vital capacity.

"ERV" means expiratory resting volume.

"CI" means confidence interval.

"PFT" means pulmonary function testing measures the function of lung capacity and lung and chest wall mechanics to determine whether or not the patient has a lung problem. Pulmonary Function Tests are commonly referred to as "PFTs". When a patient is referred for PFTs, it means that a battery of tests may be carried-out including: simple screening spirometry, static lung volume measurement, diffusing capacity for carbon monoxide, airways resistance, respiratory muscle strength and arterial blood gases.

"MEF" means maximal expiratory flow.

"Peripheral airways" means an area of the lungs primarily containing bronchi, alveoli and bronchiole, a primary site of mucus accumulation in cystic fibrosis. Large and selective depositions of an inhalable mucolytic drug in this area is eminently desirable and contributes to an efficacious treatment of severe respiratory problems due to presence of large amounts of mucus.

"Central airways" or "central lungs" means upper airways of lungs including bronchi and trachea. Current administrations of inhalable mucolytic drug deposit the drug predominantly in the central lungs.

"One breath" means a period of time when a person inhales (inspires) and exhales during a regular breathing pattern that includes inhaling and exhaling.

"Inspiration time" or "inspiration phase" means a fraction of one breath when a person inhales an air or, in this instance, an aerosolized mucolytic drug. For purposes of this invention, the aerosolized mucolytic drug is administered to a cystic fibrosis patient during the second period of the inspiration time either with a mild or moderate overpressure up to 30 mbar to force the aerosol to the peripheral airways using the AKITA® protocol and nebulizer, or as a second volume between the first and second volume of delivered air without particles using a breath actuated nebulizer and protocol.

"Expiration time" means a fraction of one breath when a person exhales the air, nitric oxide or another metabolite from the lungs. For the purposes of this invention, it is preferable that the aerosolized mucolytic drug is forced with a mild or moderate overpressure into the peripheral airways during inspiration and that it is not exhaled during expiration time or that only a small portion is exhaled.

"Bolus technique" means transportation of the aerosol containing a mucolytic drug.

"Particle-free air" means the air that does not contain any drug and is delivered before and after the aerosolized drug delivery.

"Overpressure inhalation" means inhalation with actively provided air that is preferably predefined in an airflow for a predefined time. During inspiration the patient adjusts to the inspiratory flow rate. If the patient inhales more passively an overpressure of up to maximum 30 mbar is applied during the inhalation phase to reduce the inspiratory effort. Consequently, the patient is able to inspire a larger deep inhalation volume and inhale with a slower inspiration flow rate compared to a spontaneous inhalation. Preferably the overpressure is at least about 1 mbar, 2 mbar, 3 mbar or 5 mbar. In a further embodiment the overpressure is at least about 5 mbar to 25 mbar or at least about 10 mbar to 20 mbar.

"Predominantly" means at least 80%.

"Substantially" means at least 44%.

### DETAILED DESCRIPTION OF THE INVENTION

The current invention relates to a treatment of mild or exacerbated cystic fibrosis by providing a means for delivery of a therapeutically effective amount of the aerosolized mucolytic drug dornase alpha directly to peripheral airways. The method significantly increases delivery of the aerosolized dornase alpha into alveoli and bronchioles and thereby significantly improve airway patency of the peripheral airways in cystic fibrosis.

The method utilizes nebulization devices and systems allowing individualization of a delivered volumetric flow and vaporized aerosol together with a controlled airflow and with airflow overpressure conditions into a treatment protocol suitable for cystic fibrosis patients with normal and with compromised breathing pattern. Such individualized treatment protocol provides for a shift in deposition pattern of the nebulized drug to more peripheral rather than central deposition.

For treatment of cystic fibrosis symptoms and particularly during exacerbation of cystic fibrosis symptoms, efficient delivery of dornase alpha to the patient's lung obstructed with thick mucus is extremely important. The mucus is heavily accumulated especially in the peripheral airways. It has been now found that better and much improved peripheral airways deposition can be achieved when the dornase alpha (Pulmozyme®), is delivered to the peripheral airways according to the AKITA® protocol.

The AKITA® protocol comprises treating a cystic fibrosis patient with administration of dornase alpha in an aerosol having a mass median aerodynamic diameter limited to sizes between 2 and 6 µm, preferably predominantly to 3 µm that is in such small particle sizes that are deposited in the peripheral airways rather than in the central airways. The AKITA® protocol also utilizes a more peripheral breathing pattern achieved with a slow and controlled inhalation provided by the AKITA® nebulizing system.

### I. Cystic Fibrosis and Currently Available Treatments

Respiratory disease in patients with cystic fibrosis (CF) is characterized by an abnormal production and accumulation of the epithelial lining fluid that has a high viscosity and thick mucus consistency. As a result of this accumulation, patients develop chronic airway infection and inflammation that starts early in life. The mucus in cystic fibrosis is rich in DNA released from bacteria subjected to attack by body white cells. This white cells-derived DNA contributes to abnormal visco-elasticity and thick consistency of cystic fibrosis mucus. The mucus and also purulent and infected sputum that appears during exacerbation of cystic fibrosis symptoms obstruct both the central and peripheral airways. Additionally, patients often develop exacerbation related to viral infections.

During these episodes of exacerbation, characterized by increased cough, difficulty to expectorate the mucus, loss of appetite and fatigue, the conditions of the patients deteriorate. A major challenge in the treatment of cystic fibrosis, therefore, is to facilitate a continual removal of as much mucus as possible from the lungs.

### A. Currently Available Treatments

Many treatments exist for control and management of symptoms and complications associated with cystic fibrosis. The main goal of these treatments is to prevent occurrence and recurrence of infections leading to the disease exacerbation. Because the disease itself and all associated infections leads to production of a thick mucus in the central airways and mainly in the peripheral airways, the primary aim of all cystic fibrosis treatments is to reduce the amount and thick consistency of mucus secretion, to remove as much mucus as possible and to improve airflow into and out of the lungs.

The various treatments include administration of antibiotics, steroids, bronchodilators or decongestants, a manual or mechanical drainage and, ultimately, also a lung transplantation. Treatment with antibiotics may be systemic, oral or intravenous or targeted specifically to the lungs, such as, for example, treatment of cystic fibrosis with inhalable antibiotics directed to the patient's lungs. While this is a viable treatment for cystic fibrosis, all the same it is well known that the overuse of antibiotics often leads to a development of strains of bacteria resistant to the antibiotics and often also leads to development of concurrent fungal infections. Other treatments include administration of steroids, bronchodilators and decongestants that may contribute to keep open bronchial tubes and clear secretion in the airways and in the oropharyngeal area. Still another treatment is physiotherapy that includes removal of the mucus by using manual or mechanical bronchial airway drainage techniques. This treatment is invasive, impractical and time consuming because it needs to be administered at least twice a day for 20-30 minutes. A last resort option for treatment of cystic fibrosis is lung transplantation which is, of course, a very severe invasive surgical procedure.

While the milder forms of cystic fibrosis, such as those observed in older patients, may be somehow controlled and treated with a variety of oral, systemic or inhalation therapies, severe forms of cystic fibrosis, such as those developed in small children and young patients, are very difficult to treat and manage. Moreover, the milder forms of cystic fibrosis later progresses to severe forms of this lung disease.

The exacerbations that often occur in both the adult and children patients with cystic fibrosis are difficult to treat. During these exacerbation periods, thick mucus obstructs the patient's airways resulting in breathing difficulties and threatening patient's gas exchange.

Lately developed treatments utilize mucus-thinning drugs, mucolytics, which are able to thin the thick mucus by cleaving DNA released from the bacteria in the airways. However, although the treatments with mucolytic drugs are currently the most promising treatments for cystic fibrosis, even these treatments are not without problems.

### B. Disadvantages of Latest Treatments for Cystic Fibrosis

The latest treatments for cystic fibrosis are based on finding that certain mucolytic drugs are able to chemically dissolve, destroy and/or remove mucus accumulated in the cystic fibrosis patient's lungs.

As a consequence of such mucus accumulation, patients suffering from cystic fibrosis have difficulty breathing. Accumulation of mucus in the lungs, particularly in the peripheral airways, causes partial or complete bronchial closure thereby limiting the breathing capacity. These conditions are generally treated with a variety of antibiotics administered by inhalation.

A most effective way to achieve removal of mucus from the lungs is to subject cystic fibrosis patients to an aerosolized mucolytic drug that reduces the viscosity of the mucus and sputum. Daily inhalation therapy with dornase alpha, a recombinant human DNase (rhDNase), has been shown to be effective in this manner.

RhDNase is an enzyme that cleaves and fragments extracellular DNA through hydrolysis and thereby reduces the viscosity of cystic fibrosis mucus. This, in turn, improves patient's lung function parameters, and reduces a number of pulmonary exacerbations in patients with moderate lung disease. Currently, an inhalable dornase alpha is the only known mucolytic drug having a proven efficacy in treatment of cystic fibrosis.

However, problems with the mucolytic drug treatments have been encountered and remain. Administration of the mucolytic drugs using conventional nebulizers limits the delivery of the mucolytic drug mainly to central airways and not to the peripheral airways where the main accumulation of the mucus occurs.

While the treatment with aerosolized mucolytic drug is reasonably successful in patients with moderate cystic fibrosis, its efficacy during exacerbations is insufficient. Exacerbations are characterized by the increased production of thick mucus that is difficult to expectorate. Hence more mucus is retained in the lungs and progressively blocks the airways. These exacerbations are often induced by viral infections followed by worsening of the bacterial infection followed by more severe lung inflammations. This inflammatory response is accompanied by cell death of neutrophils that sets free large amounts of free sticky DNA, further contributing to the airways blockage. The airways blockage during these exacerbation periods contributes to a severity of the disease and to a great degree of discomfort related to the patient's inability to breath.

Treatments of these exacerbation periods are proven to be difficult even with the new mucolytic drug dornase alpha administered through inhalation. These difficulties arise primarily from the currently available inefficient conventional nebulizer systems used for the delivery of the dornase alpha. Administration of this drug using the conventional inhalation methods results in an uneven and non-homogeneous distribution of the drug in the lungs resulting in only a partial relief of the cystic fibrosis symptoms. The reason for this is that currently available and used nebulizers administer a large portion of the drug primarily into the central airways and only a small portion, if any, of the drug administered into the peripheral airways where the actual accumulation of the mucus causes the most problems.

For example, the currently used nebulizer Hudson T Up-draft II with a pulmo-Aide compressor is able to deliver maximally 20% of the loaded drug, that is only 0.5 mL of the 2.5 mL filling dose of the Pulmozyme® into the patient's lungs, primarily into the central lungs.

### II. Treatment of Cystic Fibrosis

A new and improved treatment of the cystic fibrosis comprises administration of a mucolytic drug to cystic fibrosis patients as a nebulized aerosol having particle sizes of controlled homogeneous sizes corresponding to sizes of alveoli and bronchioles in the peripheral airways, using an AKITA® nebulizing system. This system permits delivery of the aerosol predominantly into the peripheral airways according to a specifically designed and individualized protocol that controls breathing pattern of a treated cystic fibrosis patient.

In one embodiment the invention therefore provides:
Dornase alpha for use in the treatment of cystic fibrosis, wherein said
   dornase alpha is administered to a cystic fibrosis patient in need thereof as an aerosol comprising said dornase alpha in a concentration of 0.75 mg/mL to 1.25 mg/mL;
wherein said aerosol is delivered from 1 mL to 5 mL of aerosolizable solution or suspension,
wherein said aerosol is aerosolized into particle sizes between 2 and 6 µm MMAD; and
wherein said aerosolized dornase alpha is administered predominantly into peripheralairways;
characterized in that the aerosol is administered with overpressure of 1 mbar to 30 mbar and under controlled conditions comprising a slow inhalation breathing pattern with a preset inspirational flow rate equal or below 20 liters/min, combined with an aerosol bolus delivery with an inspiration time comprising three periods,
wherein in the first period lasting zero seconds or from 1 millisecond to 1 second aerosolized particle free air is administered,
wherein in the second period lasting from 0.1 to 7 seconds the aerosolized dornase alphais administered, and
wherein in the third period lasting from 1 millisecond to 10 seconds aerosolized particle free air is administered.

### A. Mucolytic Drugs

The mucolytic drug useful for the purposes of this invention is dornase alpha, also known as Pulmozyme®.

RhDNase (dornase alpha) is a recombinantly produced enzyme that is an identical copy of the native human rhDNase. RhDNase is the purified glycoprotein contains 260 amino acids of approximate molecular weight of 37000 daltons. This enzyme cleaves and fragments extracellular DNA through hydrolysis and thereby reduces the viscosity of cystic fibrosis mucus. Dornase alpha hydrolyzes the DNA present in sputum/mucus of cystic fibrosis patients and reduces viscosity in the lungs, promoting improved clearance of secretions. Dornase alpha is the only registered therapeutic agent developed with this basic mechanism of action. Prior to the cloning of the human enzyme, bovine DNase I was on the market for many years, though it's utility was limited by the inherent antigenic response to a cow protein in the lungs of patients. Other DNases, such as DNase II, have some therapeutic potential, but as yet no further DNases have been found to be suitable for treatment of cystic fibrosis.

Pulmozyme® is a highly purified solution of recombinant human deoxyribonuclease I (rhDNase), an enzyme which selectively cleaves DNA. Pulmozyme® is commercially available from Genentech Inc., South San Francisco, California or from Hoffmann-La Roche Limited, Mississauga, Ontario, Canada.

For inhalation purposes, Pulmozyme® is used in concentration of 0.75 mg of the drug per 1 ml of the solvent to 1.25 mg of the drug per 1 ml of the solvent, preferably in a concentration of 1 mg of the drug per 1 mL of the solvent.

### B. Aerosol

The aerosol used for treatment of the cystic fibrosis patients comprises dornase alpha. The drug is aerosolized into particle sizes limited to between about 2 and about 6 µm, with a predominant number of at least 80% of these particles having a size of about 3 µm.

Before aerosolization, dornase alpha is dissolved in saline or sterile water in concentration of 1 mg of the drug per 1 milliliter of a solvent ((0.75mg/ml to 1.25 mg/ml, preferably 1 mg/1 mL). The solution of dornase alpha is aerosolized and delivered as an aerosol in from 1.25 mL (1.25 mg/1.25 mL) to 5 mL (5 mg/5 mL) solution.

### C. Lung Deposition

The resulting aerosols are deposited in both the central but primarily in peripheral airways using the AKITA® nebulizing system due to impaction, sedimentation and diffusion. Impaction is the main deposition mechanism in the central airways. The particles above 4 µm have higher mass velocity, and are, therefore, more likely to impact. Particles having sizes from 1 µm to about 4 µm are typically deposited by sedimentation, an important deposition mechanism in the smaller airways where air velocity is low. For particles with a diameter smaller than 0.5-1 µm, deposition is mostly due to diffusion. Deposition by Brownian motion and diffusion is the most important mechanism in the bronchioles and alveoli.

The deposition mechanisms for the drug delivery into the peripheral airways of lungs depends on the number of particles present in the aerosol and on their sizes as well as on their distribution and delivery into the peripheral airways as well as on breathing pattern of the patient.

However, the size of the particles and the normal breathing pattern alone is not sufficient to deliver a high amount of the drug to the peripheral lungs of the patient in exacerbation. Without a slow and deep breathing pattern of the patient, the particles will be deposited only according to their sizes in the upper regions of the respiratory airways. However, in cystic fibrosis patients, the preferential and desirable deposition of the drug is at sites that are partially filled and obstructed with mucus and sputum. A method is, therefore, needed that helps patient with cystic fibrosis to inhale a deep inhalation volume under a consistent slow flow rate deep into the lungs by generation an overpressure of less than 30 mbar by the inhalation device in order to push the aerosol into the peripheral airways.

The current method and devices disclosed herein provide such conditions by delivering the dornase alpha under mild or moderate overpressure and by regulating a breathing pattern during such delivery according to the AKITA® nebulizing protocol.

### D. AKITA® Nebulizing Protocol

AKITA® nebulizing protocol for treatment of cystic fibrosis comprises a preparation of aerosol of appropriate sizes to increase the efficacy of dornase alpha delivery targeted to the peripheral airways of cystic fibrosis patients, delivery of said aerosol into said peripheral airways using AKITA® nebulizer, a slow inhalation of the aerosol with aerosol bolus at start of each breath and a clinical evaluation of the patient following the inhalation treatment.

### 1. Preparation of the Aerosol

The aerosol having the optimal particle sizes for homogenous deposition of the drug in the peripheral airways that prevents high losses of drug in the oropharynx is prepared from a solution of dornase alpha (0.75mg/ml to 1.25 mg/ml, preferably 1mg/1mL) by nebulizing from about 1 to about 5 mL of said solution into an aerosol of appropriate sizes between 2 to 6 µm, preferably 3 µm, to increase the efficacy of dornase alpha delivery targeted to the peripheral airways of cystic fibrosis patients.

### 2. Delivery of the Aerosol

Delivery of the dornase alpha into peripheral airways of the lower lungs using AKITA® nebulizer system and AKITA® nebulizing protocol is achieved in less the 15 minutes, preferably in from 5 to 10 minutes and most preferably in 6 minutes. Treatment is administered several times a day, as needed, but is preferably limited to one, twice or three times a day.

### 3. Slow Inhalation with Aerosol Bolus

The patient's breathing pattern during the delivery of dornase alpha to the peripheral airways is at least as important as is the size of the aerosol particles of the nebulized dornase alpha.

The breathing pattern used to inhale the aerosol influences the deposition pattern of particles in the respiratory tract. High inspiratory flow enhances the impaction of particles, and thus enhances a more central deposition. Low inspiratory flow enables particles to penetrate more deeply into the peripheral airways. Such controlled breathing pattern is enabled by using

### AKITA® system.

An important aspect of the invention, therefore, is the ability of the AKITA® system to provide controlled conditions for patient's breathing pattern permitting a slow inhalation and, at the same time, providing aerosol bolus delivering larger dosages of the drug at start of each breath in a slow and protracted breathing inhalation maneuver.

The slow inhalation maneuver preprogrammed by AKITA® system, using the AKITA® nebulizer and delivering an aerosol comprising dornase alpha aerosolized into particle sizes predominantly of about 3 µm MMAD, enables aerosolized particles to penetrate deeply into the peripheral airways and provides a better peripheral lung deposition in patients with cystic fibrosis.

Such slow breathing pattern is limited to breathing volume of from about 50 to about 300 mL/second with inhalation volume limited to about 300 to about 2000 mL, applied with a mild to moderate overpressure up to 30 mbar.

Typically the inhalation involves a delivery of from 1 to about 5 mL, preferably from about 1.25 to about 5 mL, of dornase alpha in concentration of 0.75mg/ml to 1.25 mg/ml, preferably of 1 mg/1 mL with deposition of the dornase alpha of at least 0.8 mL, that is at least 0.8 mg of the drug that is preferably deposited into the peripheral airways.

The slow inhalation method defines a partition of one breath into two fractions, namely an inspiration time and expiration time wherein during the inspiration time a so called bolus technique is used to transport the drug containing aerosol to a predefined region in the lungs and, during the expiration time, to exhale a minimum of the drug from the lungs at end of the breath.

The method of the invention results in a highly efficient delivery of between 0.8 and 2 mg of dornase alpha, filled as from 1 to 5 ml (0.75mg/ml to 1.25 mg/ml, preferably 1mg/1mL) bolus in the AKITA® nebulizer, into the lower lungs of the patient in less than 6 to 10 minutes, on average, during the slow inhalation.

### 4. Clinical Evaluation

Clinical evaluation of the patient following the inhalation treatment with dornase alpha according to the method of the invention includes but is not limited to spirometry parameters: forced vital capacity (FVC), forced expiratory volume in one second (FEV1), forced expiratory flow after exhalation of 75% of vital capacity (FEF75), oxygen saturation parameters and profile.

### 5. Deposited Doses

The current method enables deposition of about 2-4 times more of the filling dose of the drug placed in the nebulizer in the peripheral airways of the lower lungs compared to the conventional nebulizers used for standard of treatment.

### E. AKITA® Nebulizing System

AKITA® nebulizing system (AKITA® system) provides means for controlling both the aerosolization of the drug into the particles having predominantly sizes in the range from about 2 to about 6 µm with majority of at least 44% of particles having size of about 3 µm MMAD. Using this system, particles of about 3 µm are deposited in the peripheral airways of the lower lungs, such as in alveoli or bronchioles that have sizes in this range. However, even provided that the aerosol having MMAD of these sizes may be prepared, it is still very difficult to deliver such aerosol into cystic fibrosis lungs filled with thick mucus and sputum that accumulates in the bronchioles and alveoli because the mucus and/or sputum provides a natural barrier and resistance to the deposition of the drug there. Consequently, some intervention means that would permit overcoming this problem is necessary.

AKITA® nebulizing system is equipped with a means to deliver the aerosol into such blocked alveoli and bronchioles of the peripheral airways under mild or moderate overpressure of about and up to 30 mbars. With this overpressure, the aerosolized drug is gently pushed into the patient's lungs and deposited primarily in the peripheral airways of the lower lungs. The AKITA® nebulizing system additionally provides a means for influencing a breathing pattern of the patient, which is another contributing factor to the improvement of delivery of the dornase alpha into the lungs of cystic fibrosis patient during exacerbation of the disease.

The AKITA® nebulizing system used for treatment of cystic fibrosis is therefore able to deal with all important factors that can influence treatment of cystic fibrosis, particularly during pulmonary exacerbations. The system influences the drug administration by providing the aerosol targeted primarily to the peripheral lungs delivered under mild or moderate overpressure under conditions that control a breathing pattern of the cystic fibrosis patient. The AKITA® nebulizing system is further equipped to deal with a problem of the drug delivery to both the small children having much smaller sizes of alveoli and bronchioles as well as to older children or adult patients where the sizes of the alveoli and bronchioles in the peripheral lungs is different.

Using an AKITA® treatment protocol, the dornase alpha may be prepared in an aerosol that has sizes predominantly in the range corresponding to the peripheral lungs of the small children (between 1 and 3 µm), older children and adults (about 3 µm). Each aerosol is then administered under the slight overpressure less than 30 mbar and with an individualized breathing pattern for the patient. Thus, for example, when the child is small, the aerosol MMAD particle sizes will be smaller and the inhalation volume will be adjusted to the subject's lung capacity. When the patient is adult, the aerosol will have particles sizes MMAD predominantly in the range corresponding to the sizes of the peripheral airways of the lower lungs of the adult person (about 3 µm) and delivered with a higher inhalation volume per breath especially when the disease is not in exacerbated stage.

### 1. AKITA® Inhalation System

The AKITA® inhalation system is approved for all available liquid inhalation medications. The use of a personalized smart card ensures that treatment with the AKITA® inhalation system is adjusted to the individual requirements of each patient. For a peripheral deposition, a relatively small particle size of about 3 µm and a slow, deep inhalation maneuver is used.

The AKITA® system comprises of an AKITA® compressor unit and the AKITA® nebulizer set together thereby providing a highly effective inhalation system for inhalation therapy of the peripheral airways of the lower lungs.

The AKITA® system comprises preferably the AKITA®2 compressor and the AKITA®2 nebulizer. The AKITA®2 nebulizer is able to generate particles with a MMAD from 2.0 µm to 6.0 µm and a GSD of 1.6.

**AKITA®2 nebulizer operates under following parameters:**

| | |
|---|---|
| Noise emission: | <70 dB(A) |
| Operating voltage: | 230V ±10%, 50 Hz, 0.7 A |
| Suction trigger pressure: | -1.0 to -4.0 mbar |
| Inhalation flow: | 50-300 ml/sec, adjustable, using Smartcard |
| Flow pattern: | Constant inspiration flow: 200ml/sec |
| Nebulizer pressure: | 1.5 - 2.0 bar |
| Ambient conditions: | 5 to 40°C |
| | 10 to 95% relative humidity |
| | 600 to 1100 hPa atmospheric pressure |

### 1. Particle Sizes of the Aerosol

The AKITA® nebulizing system provides an aerosol having the optimal particle sizes for homogenous deposition in the peripheral airways of the lower lungs that prevents high losses of drug in the oropharynx as well as losses in upper lungs.

The system provides an aerosol having sizes of aerosolized particles corresponding substantially to a size of the alveoli and bronchiole. The right particle size for targeting the alveoli and bronchiole is between 2 and 6 microns, preferably about 3 µm. Particles larger than 3 µm are selectively deposited in the more central and upper lungs, namely bronchi and trachea and in the mouth and throat, i.e. oropharyngeal area.

Consequently, the method provides for aerosol to be limited to particle sizes between 2 and 6 microns, preferably to particle sizes of about 3 µm MMAD with geometric standard deviation (GSD) of less than 2.5, preferably GDS of about 1.6.

The age of the patient is important when deciding on the particle sizes of the aerosol for dornase alpha delivery to the cystic fibrosis patients because the airway size has an important effect on the deposition pattern of the inhaled aerosol. The airway diameter in children and infants is smaller than in adults. As a result, aerosol particles tend to be deposited more in central airways in children relative to the deposition pattern observed in adults making the treatment even more difficult. It is therefore important to regulate the particle sizes also according to the age of the patient.

Furthermore, edema of the airway walls, mucus, sputum or pulmonary bronchoconstriction cause narrowing of the airway diameter and, consequently, the inhaled aerosol is largely deposited in upper and central rather than peripheral airways. This is often observed in cystic fibrosis patients during the exacerbation period, when there is often increased airway obstruction due to infections, inflammations and more mucus and sputum. Consequently, any inhalation treatment shifts the deposition to a more central deposition pattern.

### 2. Delivery of the Aerosol under Overpressure

As discussed already above, delivery of the dornase alpha by an aerosol without any enhancement results in more central than peripheral airways. Such enhancement is provided for by the instant method by delivering the drug containing aerosol under mild or moderate overpressure.

The system provides means to deliver the dornase alpha containing aerosol under overpressure no higher than 30 mbar. Such mild to moderate overpressure allows the aerosol to be actively forced to the peripheral airways of the lower lungs even when filled with mucus or sputum without causing damage to the lungs.

Such overpressure is achieved with an AKITA® compressor with or without pump unit attached to the AKITA® nebulizer where such unit is optionally further equipped with a timer so that the overpressure period is limited strictly to a fraction of the inspiration time when the dornase alpha aerosol is delivered and, moreover, AKITA® system has a safely means shutting off the pressure at 30 mbar.

In one embodiment, the overpressure is initiated by a patient's inspiration time breathing. When the patient inspires with overpressure, the patient's breathing effort is reduced and the patient is able to breath in a deeper and slower breathing pattern. That makes a great difference when compared to a spontaneous inhalation administered without overpressure. The overpressure is preset and regulated according to the treatment protocol.

During the inhalation, the AKITA® nebulizing system provides an overpressure of up to 30 mbar under which the aerosol is administered. Such overpressure allows preferable deposition of the aerosolized drug in the peripheral airways of the lower lungs and also prevents the aerosol removal during expiration because during expiration, the overpressure is not applied and the patient thus exhales normally, without any airflow or pressure being applied.

### 3. Bolus Technique

The AKITA® nebulizing system and a method for use thereof defines a partition of one breath into two fractions, namely an inspiration time and expiration time wherein during the inspiration time a bolus technique is used to transport the drug containing aerosol to a predefined region, in this case to the peripheral airways of the lower lungs, and during the expiration time, to expire a minimum of the drug from the lungs.

In some embodiments of the bolus technique, the inspiration time may be further divided into subfractions where the particle free air is delivered before and after the aerosol delivery of the dornase alpha.

### 4. Delivery Time

The system provides for shorter delivery time than conventional nebulizer for the same drug amount deposited in the lungs. Typically, the delivery of the 2.5 mL (2.5 mg/2.5 mL) of the dornase alpha aerosol takes approximately 20 minutes using a conventional nebulizer and results in deposition of only about 0.5 mL (0.5 mg) of dornase alpha. The current method provides for a deposition of more than 0.8 mL of the 1.25 and preferably about at least 2 mL of the filled 5 mL, of dornase alpha containing aerosol in the lungs in less than 15 minutes, preferably in less than 6 minutes, resulting in delivery of more than 0.8 mg of dornase alpha and preferably up to about 2 mg of dornase alpha per one treatment.

### III. Treatment Protocol for Treatment of Cystic Fibrosis

The actual treatment protocol (AKITA® protocol) for treatment of cystic fibrosis according to the invention consists of several steps that need to be undertaken.

### A. Inhalation System for Control of Breathing Pattern

When the AKITA® protocol is selected for treatment, the patient is provided with the AKITA® nebulizing system, as described below.

The dornase alpha in the predetermined volume of about 1 to about 5 mL containing0.75mg/ml to 1.25 mg/ml, preferably 1 mg/1 mL of the drug is filled into the nebulizer. For example, 1.25 mL of dornase alpha is filled in the nebulizer in form of an aqueous suspension or in form of 1 to 5 ml solution that contains dornase alpha in concentration 1 mg/1 mL.

The nebulizer is directly connected with the mouthpiece that is further equipped with pressure sensor connected with a compressor. Inhalation period (inspiration time) is preset to a pattern comfortable for a patient, for example, from 1 to about 10, adjusted to the patient's lung capacity, of inspiration time. When the inspiration time is not preset, patient's own breathing rhythm controls the inspiration time.

When the patient inhales from the mouthpiece, the pressure sensor responds and starts inhalation by providing a positive overpressure or opening of an inspiration valve. The nebulizer, or an aerosol system, is supplied with compressed air overpressure of up to maximum 30 mbar and the dornase alpha is aerosolized and discharged as an aerosol at a preselected flow rate of about 50-300 mL/sec and overpressure less than 30 mbar. The overpressure lasts for the entire inspiration time. When the inspiration time is preselected for a certain period of time, the overpressure is automatically stopped or shut off at the end of this period because the compressed air supply is interrupted at the end of the inspiration time. After a period allocated for exhaling, the process is repeated on and off for the entire period of inhalation, preferably for less than 6 minutes. During the inhalation time, the whole dose is preferably aerosolized with only some small residue remaining in the nebulizer.

Electronic equipment that may be attached to the nebulizer permits recordation of the inhalation process, storing all records regarding the dose, time, air flow and overpressure for further optimization of the treatment.

When this method of delivery is selected, during the inspiration time the aerosolized dornase alpha is forced under the overpressure into the peripheral airways of the lower lungs. When the overpressure is withdrawn and the patient exhales, the drug forced into the lower lungs is not easily displaced and remains there resulting in substantially higher deposition of the dornase alpha and therefore stronger mucolytic action in the peripheral lungs than would happen with a normal breathing pattern without overpressure.

During the exhalation time, the small amount of the drug that is exhaled is the one that was in the upper lungs at the last moment of the inspiration time. Some fraction of this small amount may be deposited in the upper lungs or oropharyngeal area but most of the drug is exhaled to the outside of the mouth.

When the above treatment was tested on 34 patients in two independent cystic fibrosis centers in a randomized, controlled double blind clinical trial with inhalable dornase alpha (2 mg/2mL) aerosol having particle sizes of 3 µm MMAD, for seven days and compared to a group where the particle sizes were 6 µm, as described in Example 1, such treatment resulted in significant improvement of FEF by at least 10%.

### B. Breath Actuated Treatment Protocol

The second method for treatment of cystic fibrosis comprises use of a nebulizing system that is actuated by patient's breathing and comprises the use of a breath actuated nebulizer.

This nebulizer permits depositing aerosolized particles to specific areas of the lung by regulating aerosolization parameters of the device and by instituting a three prong inspiration time delivery.

Using breath actuated nebulizer system, the dornase alpha, in the predetermined amount and volume, as already described above, is filled into the drug cartridge connected with the nebulizer that includes the mouthpiece and a spirometer.

The predefined volume of aerosolized particles is delivered into the flow path through which the patient is inhaling. Inhalation time is preset to comprise a three predefined periods.

The first predefined time period is for delivery of aerosol particle free air into the lungs at a flow rate that is also preset.

The second predefined period is for delivery of a predefined volume of aerosolized particles of the dornase alpha, also at a preset flow rate.

The third predefined period is for delivery of the second predefined time period of a particle free air.

Optionally, the first time period can be set to zero seconds, meaning that the aerosolization will start immediately without the delivery of the particle free air.

During the inhalation, patient is instructed to begin inhalation and during each inspiration time, the three (or two) predetermined periods are repeated. At the end of the second particle free period, that is after the second predefined period, a patient is instructed to stop inhaling and exhale. The reason for the second predefined time period of aerosol particle free air delivery into the lungs at a flow rate within the preset flow rate range is to move the aerosolized particles out of the upper airway region. In that way the upper airway region (mouth, throat, oropharynx, larynx and trachea) is emptied from remaining aerosol particles and the deposition of the drug in this region is reduced.

The method additionally comprises a step of detecting when the subject is inhaling through the flow path and may further comprise steps of measuring and adapting the first, the second and the third predefined time period and/or the predefined volume of aerosolized particles to patient's health parameters.

The method determines optimal time intervals for administration of the first particle-free air, for administration of an aerosolized inhalable dornase alpha and for administration of the second particle free air, wherein the cumulative time for these three time intervals corresponds to one inspiration time. The time for each of the interval corresponds to from about 1 msec to about 10 sec, preferably from about 200 msec to about 5 seconds, and may be the same or different for each interval.

The flow rate is a predetermined fixed flow rate, wherein the first predefined particle free air volume is up to about 0.15 liters, the predefined volume of aerosolized particles is up to about 3 liters and the second predefined particle free air volume is up to about 0.5 liters.

The nebulizer used for this method is equipped to detect when the subject is inhaling through the flow path and prevent flow through the flow path after providing the second predefined time period of aerosol particle free air.

### IV. Devices and Properties Thereof

Devices suitable for practicing the current invention have to have certain properties that meet the criteria for delivery of inhalable dornase alpha to the peripheral airways according to the invention.

### A. Device for Control of Breathing Pattern

The device for control of breathing pattern is suitable for practicing the current invention is an inhalation system that comprises a compressor-driven jet nebulizer that controls the patient's breathing pattern during the inspiration phase. This system is highly effective for inhalation therapy requiring deposition of the aerosol into the lower lungs. During the inhalation, the system controls number of breaths, flow rate and inspirational volume. This ability to control these three parameters assures that the patient is given a correct dose.

The system further comprises an electronic means for individual personalization of a treatment protocol. The treatment protocol includes such parameters as individual's lung function measurements, optimum breathing pattern, desired drug dose to maintain or restore patients vital capacity (VC), expiratory resting volume (ERV) and forced expiratory volume per one second (FEV1). These parameters are individualized and stored on individual electronic record, called Smart Card. The electronic records not only store the information for a treatment protocol and transfer this information to the system during treatment but also record and store the information for each of the treatments and show a possible error.

The Smart Card system may hold more than one treatment configuration and is fully encrypted. The Smart Card system is disclosed in the co-pending US patent application 2001/0037806 A1, published on November 8, 2001. The same or similar nebulizing system is disclosed in the US patent 6,606,989 and is commercially available from Activaero GmbH, Gemünden (Wohra), Germany, under the trade name AKITA® Inhalation System.

A similar but modified device for the inhalation system further comprises, as a core element, a circular perforated membrane, that may be set to vibrate by a piezo-electric actuator. The vibrating motion of the membrane generates an alternating pressure that forces the nebulizing solution through a microarray of perforation in the membrane thus creating a fine aerosol having defined particle sizes. This system is similarly equipped with electronic means comprising the Smart Card, as described above. This system is commercially available from Activaero GmbH, Gemünden (Wohra), Germany, under the trade name AKITA² APIXNEB Inhalation System.

Another device comprising modifications of the inhalation system that can be used for practicing the current invention is the nebulizer that is triggered by the negative trigger pressure detected by a pressure sensor. This nebulizer comprises a compressor that provides a constant inhalation flow rate of 12 liters/minute during inspiration and has a controlled flow, volume and nebulization timing. The Smart Card settings include inhalation volume, inhalation time per breath, nebulization time per one breath. This system is commercially available from Activaero GmbH, Gemünden (Wohra), Germany, under the trade name AKITA JET Inhalation System.

Other inhalation devices and systems that may be conveniently used or modified for use by the current invention are disclosed in the US patents 6,401,710 B1, 6,463,929 B1, 6,571,791 B2, 6,681,762 B1 and 7,077,125 B2 or in published applications 2006/0201499 A1 and 2007/0006883 A1.

### B. Breath Actuated Nebulizer Device

Another type of device suitable for practicing the current invention is a breath actuated nebulizer. This nebulizer is characterized by a passive flow and active volume control. Typically, it comprises a single use aerosol generator and a multi-use control device.

The device consists of an inhaler that is connected with a control unit. Inhaler itself is connected with nebulizer where the inhalable dornase alpha is nebulized into predetermined particles having sizes predominantly in the range from about 2 to about 6 µm, preferably about 3 µm MMAD, using an aerosol generator. The filling volume of the nebulizer is approximately 4 ml. The aerosol generator is activated by pressure detection and is only activated during inspiration phase when the patient is inhaling the aerosolized dornase alpha. The pressure detection is controlled electronically.

This device is further equipped with means to permit administration of particle-free air, to permit the administration of an aerosolized inhalable dornase alpha, and to permit the second administration of the particle free air, each for a preselected time and volume, wherein the cumulative time for these three time intervals correspond to one inspiration time. The time for each of the interval corresponds to from about 1 msec to about 10 sec, preferably from about 200 msec to about 5 seconds.

The inhaler has an integrated flow and volume limited to about 15 liters/minute flow at a pressure of about 10 mbar or lower. When the underpressure at the mouthpiece is below 5 mbar, the flow rate is limited by a mechanical valve. The mechanical valve regulates the flow rate by a adjusting the cross section area. The unit is preset to a volume per one breath. One breath is set to be a time when one inspiration and one expiration occur. After each inspiration time, the inspiration flow is blocked and expiration allowed. The inspiration flow is restored again for the next inspiration time during the next breath.

This device has various electronic components that permit its preprogramming and individualization meeting requirements of the individual asthmatic patients.

The modified device and method for its use is disclosed in the U.S. application Ser. No.: 12/204,037.

### V. Advantages of the Treatment of Cystic Fibrosis

The method for treatment of cystic fibrosis, particularly its severe exacerbations stages, according to the current invention, provides several advantages over the currently available treatments.

The method for treatment of cystic fibrosis according to the current invention provides a substantial improvement in cystic fibrosis lung functions compared to the currently available conventional treatments, as determined by the measurements of vital capacity of the peripheral airways using maximal expiratory flow (MEF 25 or MEF 75), forced expiratory volume (FEV), forced expiratory flow rate (FEF) or forced vital capacity (FVC).

The method for treatment of cystic fibrosis according to the current invention results in a faster improvement of clinical symptoms of cystic fibrosis due to a deposition of larger amounts of dornase alpha in the peripheral lungs, leading to higher fragmentation of the DNA and higher degradation and removal of mucus and/ or sputum from the patient's lungs.

The method for treatment of cystic fibrosis according to the current invention allows a deposition of high doses of inhalable dornase alpha in the peripheral airways of the lower lungs of cystic fibrosis patients, with a concurrent reduction in central and upper lung deposition and in reduction of oropharyngeal side effects, because it assures that the aerosolized particles of the drug are deposited deep into the peripheral airways of the lower lungs due to the slow and regulated breathing pattern.

The method further provides an aerosol having the optimal particle sizes for homogenous deposition of the drug in the peripheral airways of the lower lungs that prevents high losses of drug in the oropharynx.

The method for treatment of cystic fibrosis according to the current invention also provides for administration of the aerosol, during inhalation, under a mild or moderate controlled overpressure to allow preferable deposition of the aerosolized drug into the peripheral airways of the lower lungs and prevent exhalation of aerosol during the exhalation phase

### Example 1

### Efficacy of Dornase Alpha Delivery

Efficacy of RhDNase targeted to the peripheral airways in CF patients in stable condition was studied in a randomized controlled clinical trial under the following conditions.

A randomized controlled, double blind, clinical trial was performed in three cystic fibrosis centres. The trial included 49 cystic fibrosis patients who were already on maintenance use of DNase at inclusion of the study.

After screening, patients were randomized to dornase alpha (Pulmozyme®, 1mg/1mL) targeted to peripheral airways or to central airways once daily for 28±2 days, using the AKITA® APIXNEB nebulizer.

Aerosol for peripheral setting: MMAD 4.0 µm, slow inhalation of 200 ml/sec with aerosol bolus at start of each breath.

Aerosol for central setting: MMAD 6.0 µm, normal inhalation with aerosol bolus in middle of each breath.

Spirometry was performed at inclusion, after 14±2 days of treatment and after 28±2 days of treatment.

Primary endpoint measured was FEF75. Secondary endpoints measured were FEV1, FVC. In a subgroup of patients LCI (lung clearance index) was measured, since this measurement was only available in 1 of the 3 centers. For safety monitoring patients recorded symptoms in a daily diary during the 4 weeks of treatment.

Statistical methods used were: Analyses of between-group comparisons regarding change in lung function (FVC, FEV₁, FEF₇₅, LCI) were performed using analysis of covariance (ANCOVA) with adjustment for baseline values and center. Differences were expressed as change from baseline sds for FVC, FEV₁ and FEF₇₅. Spirometry variables were expressed as Z-scores (sds) and % predicted using the reference values by Stanojevic et al.; American Journal of Respiratory and Critical Care Medicine Vol 177. pp. 253-260, (2008) for FVC, FEV₁ and MMEF. Since these reference equations do not include data for FEF₇₅, we used the reference values by Zapletal et al., Lung function in children and adolescents. Methods, reference values. Basel: Karger, 1987 to calculate Z-scores and % predicted for FEF₇₅. Data were analysed on an intention-to-treat basis. The primary analysis on FEF₇₅ was repeated in a per protocol analysis, excluding patients who did not complete the 4 weeks of study treatment and patients who had a daily dose compliance (DDC) of <70%).

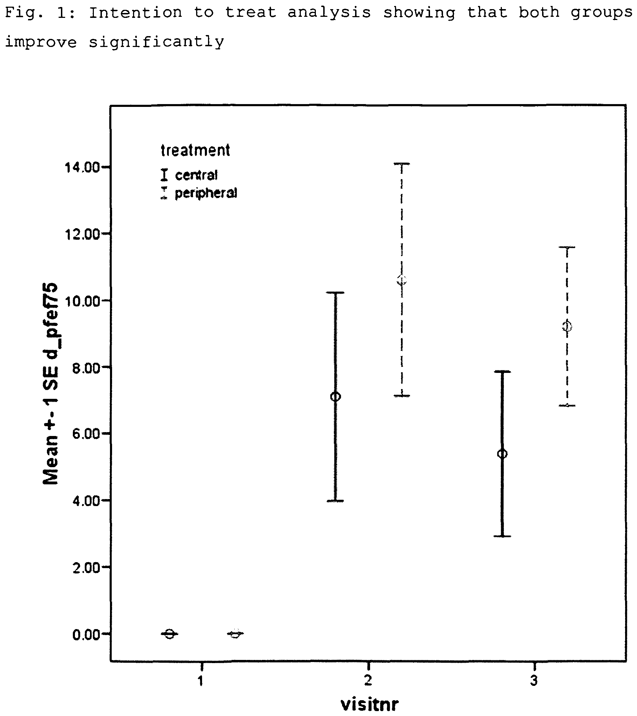

Results of the intention to treat analysis obtained in this study show that both groups improve significantly: FEF₇₅ increased 9.2% in the peripheral group (p < 0.001) and 5.2% in the central group (p = 0.030) at visit 3. The difference between the groups is not statistically significant: Difference = 3.6%, 95% CI: -2.87 - 10.14 (p = 0.27) as it is shown in Figure 1.

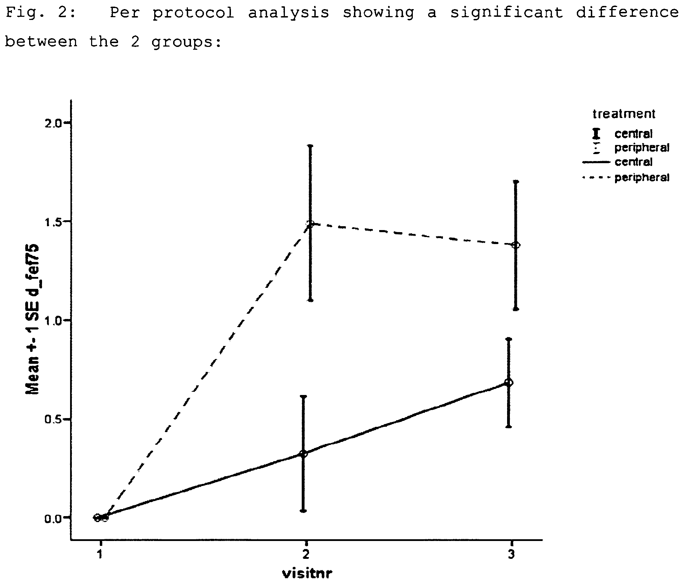

The per protocol analysis showed a significant difference between the 2 groups: At visit 2 there is a significant difference: peripheral is 1.18 SDS higher than central, p = 0.023. At visit 3 there is a trend: Peripheral is 0.73 SDS higher than central (p = 0.056) as it is shown in Figure 2.

Safety monitoring using the symptom scores from the diaries did not show any Serious Adverse Events (SAE's). There was no difference in number of Adverse Events (Ae's) between the two treatment groups. All AE's were mild and were symptoms that can be expected in CF (e.g. cough, common cold, stomach ache, runny nose, etc.).

## Claims

1. Dornase alpha for use in the treatment of cystic fibrosis, wherein said dornase alpha is administered to a cystic fibrosis patient in need thereof as an aerosol comprising said dornase alpha in a concentration of 0.75 mg/mL to 1.25 mg/mL;
wherein said aerosol is delivered from 1 mL to 5 mL of aerosolizable solution or suspension,
wherein said aerosol is aerosolized into particle sizes between 2 and 6 µm MMAD; and wherein said aerosolized dornase alpha is administered predominantly into peripheral airways;
**characterized in that** the aerosol is administered with overpressure of 1 mbar to 30 mbar and under controlled conditions comprising a slow inhalation breathing pattern with a preset inspirational flow rate equal or below 20 liters/min, combined with an aerosol bolus delivery with an inspiration time comprising three periods,
wherein in the first period lasting zero seconds or from 1 millisecond to 1 second aerosolized particle free air is administered,
wherein in the second period lasting from 0.1 to 7 seconds the aerosolized dornase alphais administered, and
wherein in the third period lasting from 1 millisecond to 10 seconds aerosolized particle free air is administered.

2. Dornase alpha for use as defined in claim 1, wherein the patient is suffering from cystic fibrosis exacerbations.

3. Dornase alpha for use as defined claim 1 or 2, wherein said aerosol is administered during the inhalation phase of the patient with an overpressure of 5 to 25 mbar.

4. Dornase alpha for use as defined in claim 3, wherein said aerosol is administered during the inhalation phase of the patient with an overpressure of 10 to 20 mbar.

5. Dornase alpha for use as defined in any preceding claim, wherein said aerosol has a mass median aerodynamic diameter (MMAD) of 3 µm and/or a geometric standard deviation (GSD) of less than 2.5, preferably of about 1.6.

6. Dornase alpha for use as defined in claims 1 to 5, wherein said aerosol has a mass median aerodynamic diameter (MMAD) from 1 to 3 µm.

7. Dornase alpha for use as defined in any preceding claim, wherein during the first period aerosolized particle free air is administered at a preset volume of less than 150 mL in 0.5 seconds time.

8. Dornase alpha for use as defined in claim 7, wherein during the second period the aerosolized dornase alpha is administered at a volume of from 200 mL to 2000 mL or in a preset time of from 1 second to 7 seconds.

9. Dornase alpha for use as defined in claim 8, wherein during the third period aerosolized particle free air is administered at a preset volume from 200 mL to 500 mL in 0.3 seconds to 3.0 seconds time.

10. Dornase alpha for use as defined in any preceding claim, wherein said aerosol is administered once, twice or three times a day.

11. Dornase alpha for use as defined in any preceding claim, wherein said aerosol is administered by a breath-actuated inhalation device.

12. Dornase alpha for use as defined in any preceding claim, wherein said aerosol is administered by an inhalation device.

## Patentansprüche

1. Dornase alfa zur Verwendung bei der Behandlung von Mukoviszidose, wobei Dornase alfa einem Mukoviszidose-Patienten, der sie benötigt, als ein Aerosol verabreicht wird, das die genannten Dornase-alfa-Konzentrationen von 0,75 mg/mL bis 1,25 mg/mL umfasst;
Wobei das Aerosol als 1 mL bis 5 mL einer aerosolisierbaren Lösung oder Suspension abgegeben wird,
wobei das Aerosol in Partikelgrößen zwischen 2 und 6 µm MMAD aerosoliert wird; und wobei die aerosolierte Dornase alfa überwiegend in die peripheren Atemwege abgegeben wird;
**dadurch gekennzeichnet, dass** das Aerosol mit einem Überdruck von 1 mbar bis 30 mbar und unter kontrollierten Bedingungen mit einem langsamen Inhalationsmuster mit einem voreingestellten Inspirationsdurchfluss gleich oder kleiner als 20 Liter/min verabreicht wird, kombiniert mit einer Aerosol-Bolusabgabe mit einer Inspirationszeit von drei Perioden,
wobei in der ersten Periode von null Sekunden oder von 1 Millisekunde bis 1 Sekunde aerosolierte partikelfreie Luft verabreicht wird,
wobei in der zweiten Periode von 0,1 bis 7 Sekunden die aerosolierte Dornase alfa verabreicht wird, und
wobei in der dritten Periode von 1 Millisekunde bis 10 Sekunden aerosolierte partikelfreie Luft verabreicht wird.

2. Dornase alfa zur Verwendung wie in Anspruch 1 definiert, wobei der Patient an Mukoviszidose-Exazerbationen leidet.

3. Dornase alfa zur Verwendung wie in Anspruch 1 oder 2 definiert, wobei das Aerosol während der Inhalationsphase des Patienten mit einem Überdruck von 5 bis 25 mbar verabreicht wird.

4. Dornase alfa zur Verwendung wie in Anspruch 3 definiert, wobei das Aerosol während der Inhalationsphase des Patienten mit einem Überdruck von 10 bis 20 mbar verabreicht wird.

5. Dornase alfa zur Verwendung wie in einem der vorhergehenden Ansprüche definiert, wobei das Aerosol einen massenmittleren aerodynamischen Durchmesser (MMAD) von 3 µm und/oder eine geometrische Standardabweichung (GSD) von weniger als 2,5, vorzugsweise von etwa 1,6, aufweist.

6. Dornase alfa zur Verwendung wie in den Ansprüchen 1 bis 5 definiert, wobei das Aerosol einen massenmedianen aerodynamischen Durchmesser (MMAD) von 1 bis 3 µm aufweist.

7. Dornase alfa zur Verwendung wie in irgendeinem vorhergehenden Anspruch definiert, wobei während der ersten Periode aerosolierte partikelfreie Luft mit einem vorgegebenen Volumen von weniger als 150 mL in 0,5 Sekunden Zeit verabreicht wird.

8. Dornase alfa zur Verwendung wie in Anspruch 7 definiert, wobei während der zweiten Periode die aerosolierte Dornase alfa in einem Volumen von 200 mL bis 2000 mL oder in einer vorgegebenen Zeit von 1 Sekunde bis 7 Sekunden verabreicht wird.

9. Dornase alfa zur Verwendung wie in Anspruch 8 definiert, wobei während der dritten Periode aerosolierte partikelfreie Luft mit einem vorgegebenen Volumen von 200 mL bis 500 mL in 0,3 Sekunden bis 3,0 Sekunden Zeit verabreicht wird.

10. Dornase alfa zur Verwendung wie in einem der vorhergehenden Ansprüche definiert, wobei das Aerosol ein-, zwei- oder dreimal täglich verabreicht wird.

11. Dornase alfa zur Verwendung wie in einem der vorhergehenden Ansprüche definiert, wobei das Aerosol durch eine atemgesteuerte Inhalationsvorrichtung verabreicht wird.

12. Dornase alfa zur Verwendung wie in irgendeinem der vorhergehenden Ansprüche definiert, wobei das Aerosol von einem Inhalationsgerät verabreicht wird.

## Revendications

1. Dornase alpha destinée à être utilisée dans le traitement de la mucoviscidose, ladite dornase alpha étant administrée à un patient atteint de mucoviscidose qui en a besoin sous forme d'un aérosol comprenant ladite dornase alpha en une concentration de 0,75 mg/ml à 1,25 mg/ml ;
ledit aérosol étant fourni à partir de 1 ml à 5 ml de solution ou suspension pouvant être aérosolisée,
ledit aérosol étant aérosolisé à des tailles de particules comprises entre 2 et 6 µm en termes de MMAD ; et
ladite dornase alpha aérosolisée étant administrée principalement dans les voies respiratoires périphériques ;
**caractérisée en ce que** l'aérosol est administré avec une surpression de 1 mbar à 30 mbar et dans des conditions contrôlées comprenant un mode de respiration à lente inspiration avec un débit d'inspiration préréglé inférieur ou égal à 20 litres/min, combiné avec un apport de bolus d'aérosol avec un temps d'inspiration comprenant trois périodes,
dans la première période durant zéro seconde ou de 1 milliseconde à 1 seconde de l'air exempt de particules aérosolisées étant administré,
dans la deuxième période durant de 0,1 à 7 secondes la dornase alpha aérosolisée étant administrée et
dans la troisième période durant de 1 milliseconde à 10 secondes de l'air exempt de particules aérosolisées étant administré.

2. Dornase alpha destinée à être utilisée comme défini dans la revendication 1, le patient souffrant d'exacerbations de mucoviscidose.

3. Dornase alpha destinée à être utilisée comme défini dans la revendication 1 ou 2, ledit aérosol étant administré pendant la phase d'inspiration du patient avec une surpression de 5 à 25 mbar.

4. Dornase alpha destinée à être utilisée comme défini dans la revendication 3, ledit aérosol étant administré pendant la phase d'inspiration du patient avec une surpression de 10 à 20 mbar.

5. Dornase alpha destinée à être utilisée comme défini dans une quelconque revendication précédente, ledit aérosol ayant un diamètre aérodynamique médian en masse (MMAD) de 3 µm et/ou un écart-type géométrique (GSD) inférieur à 2,5, de préférence d'environ 1,6.

6. Dornase alpha destinée à être utilisée comme défini dans les revendications 1 à 5, ledit aérosol ayant un diamètre aérodynamique médian en masse (MMAD) de 1 à 3 µm.

7. Dornase alpha destinée à être utilisée comme défini dans une quelconque revendication précédente, pendant la première période de l'air exempt de particules aérosolisées étant administré à un volume préréglé inférieur à 150 ml sur une durée de 0,5 seconde.

8. Dornase alpha destinée à être utilisée comme défini dans la revendication 7, pendant la deuxième période la dornase alpha aérosolisée étant administrée à un volume de 200 ml à 2000 ml ou sur une durée préréglée de 1 seconde à 7 secondes.

9. Dornase alpha destinée à être utilisée comme défini dans la revendication 8, pendant la troisième période de l'air exempt de particules aérosolisées étant administré à un volume prédéfini de 200 ml à 500 ml sur une durée de 0,3 seconde à 3,0 secondes.

10. Dornase alpha destinée à être utilisée comme défini dans une quelconque revendication précédente, ledit aérosol étant administré une fois, deux fois ou trois fois par jour.

11. Dornase alpha destinée à être utilisée comme défini dans une quelconque revendication précédente, ledit aérosol étant administré par un inhalateur activé par la respiration.

12. Dornase alpha destinée à être utilisée comme défini dans une quelconque revendication précédente, ledit aérosol étant administré par un inhalateur.
